# EUROPÄISCHE PATENTSCHRIFT

(11) **EP 2 134 247 B1**
(45) Veröffentlichungstag und Bekanntmachung des Hinweises auf die Patenterteilung: **06.12.2017**
(21) Anmeldenummer: 08734833.0
(22) Anmeldetag: 28.03.2008
(51) Int. Cl.: A61B 3/10, G01B 9/02, G01B 11/06

(54) **VERFAHREN ZUR ACHSLÄNGENMESSUNG MIT ERWEITERTER MESSFUNKTION IM VORDEREN AUGENABSCHNITT**
METHOD FOR AXIAL LENGTH MEASUREMENT HAVING EXPANDED MEASURING FUNCTION IN THE ANTERIOR EYE SEGMENT
PROCÉDÉ DE MESURE DE LA LONGUEUR D'UN AXE À FONCTION DE MESURE ÉLARGIE DANS LA PARTIE AVANT DE L'OEIL

(30) Priorität: 13.04.2007 DE 102007017599
(43) Veröffentlichungstag der Anmeldung: 23.12.2009
(73) Patentinhaber: Carl Zeiss Meditec AG, 07745 Jena (DE)
(72) Erfinder: BERGNER, Roland, 07745 Jena (DE); KOSCHMIEDER, Ingo, 07743 Jena (DE); VON BÜNAU, Rudolf, 07749 Jena (DE); DREHER, Claus, 99423 Weimar (DE)
(86) Internationale Anmeldenummer: PCT/EP2008/002457
(87) Internationale Veröffentlichungsnummer: WO 2008/125198

(56) Entgegenhaltungen:
- EP-A1- 1 430 829
- WO-A-2005/077256
- DE-A1- 19 857 001
- DE-A1-102005 026 371
- US-A- 5 493 109
- US-B1- 6 779 891
- IZATT J A ET AL: "MICROMETER-SCALE RESOLUTION IMAGING OF THE ANTERIOR EYE IN VIVO WITH OPTICAL COHERENCE TOMOGRAPHY" ARCHIVES OF OPHTHALMOLOGY, XX, XX, Bd. 112, 1. Dezember 1994 (1994-12-01), Seiten 1584-1589, XP001040826 ISSN: 0003-9950
- KOHNEN ET AL: "Internal anterior chamber diameter using optical coherence tomography compared with white-to-white distances using automated measurements" JOURNAL CATARACT AND REFRACTIVE SURGERY, SURGERY, FAIRFAX, VA, Bd. 32, Nr. 11, 1. November 2006 (2006-11-01), Seiten 1809-1813, XP005841425 ISSN: 0886-3350

## Beschreibung

Die vorliegende Erfindung betrifft eine Lösung zur Messung geometrischer, zur Berechnung der Brechkraft von Intraokularlinsen erforderlicher Parameter im Auge. Diese unter dem Begriff Biometrie bekannten Messungen sind insbesondere auch im Hinblick auf die Berechnung von Intraokularlinsen nach früherer refraktiver Hornhautchirurgie von Bedeutung.

Nach dem bekannten Stand der Technik sind dazu zahlreiche Lösungen bekannt, die auf verschiedenen technischen Grundlagen basieren.

PCI ist heute in Form des IOLMaster als Goldstandard für hochgenaue Achslängenmessung etabliert. Zusätzlich zur Achslänge (AL) liefert der IOLMaster drei weitere Messgrößen - zentrale Radien der Hornhaut-Vorderfläche (K), Vorderkammertiefe (ACD), und Limbusdurchmesser (WTW), die in unterschiedlicher Kombination in moderne IOL-Berechnungsformeln eingehen. So nutzt die Haigis-Formel die Eingangsgrößen AL, K, und ACD; die Holladay-II-Formel nutzt zusätzlich den Parameter WTW.

Hierzu zeigt **Figur 1** den prinzipiellen Aufbau eines IOLMasters gemäß DE 198 57 001 A1. Zur Vermessung der Achslänge AL wird hierbei das von der Laserdiode **2** ausgesendete Licht über ein Michelson-Interferometer **3** und einen Strahlteilerwürfer **8** auf das Patientenauge **1** abgebildet. Das Michelson-Interferometer **3** besteht dabei aus einem feststehenden Referenzarm mit Tripelprisma **4,** einem verstellbaren Messarm mit verschiebbarem Tripelprisma **5** und einem Strahlteilerwürfel **6** zur Überlagerung der beiden reflektierten Strahlungsanteile. Von einem Wegmesssystem **7** werden die Weglängenunterschiede ermittelt. Die von Hornhaut und Netzhaut des Auges **1** reflektierten Teilstrahlen überlagern sich und werden über Strahlteilerwürfel **8,** Achromat **9** und Strahlteiler **10** auf eine Avalanche-Photodiode **10** abgebildet. Zur Beobachtung des Auges **1** und der entstehenden Reflexe wird ein Teil des vom Auge **1** kommenden Lichtes auf einen Empfänger **12.** Weitere notwendige Parameter des Auges zur Berechnung der erforderlichen IOL werden mit Hilfe der Keratometer-Messung (Lichtquellen **13** und **15** mit den Beleuchtungsoptiken **14** und **16**) und der VKT-Messung (Lichtquelle **17** mit Beleuchtungsoptik **18**) bestimmt.

Verschiedene Veröffentlichungen postulieren, dass mit Hilfe weiterer Messgrößen, wie z.B. der Dicke der natürlichen Linse, möglicherweise auch der Linsenradien, eine genauere Vorhersage der postoperativen IOL-Position möglich werden sollte. Das wiederum sollte eine genauere Berechnung der IOL-Stärke und damit eine verringerte Streuung der post-operativen Refraktionsergebnisse erlauben.

Auch kann die Bestimmung der Achslänge durch eine Messung axialer Teilstrecken wie Linsendicke und Hornhautdicke prinzipiell verbessert werden, da sich die Brechzahlen der unterschiedlichen Medien im Auge unterscheiden, üblicherweise aber bei der Umrechnung von optischer Weglänge zu physikalischer Weglänge durch eine einzige effektiven Brechzahl wiedergegeben werden. Eine Berücksichtigung der Teilstrecken und ihrer unterschiedlichen Brechzahlen ermöglicht mithin eine genauere Bestimmung der Achslänge.

Weiterhin verfolgen mehrere Autoren einen Raytracing-Ansatz, der mit Hilfe der Strahldurchrechnung die nicht-paraxiale Abbildung in die Optimierung der IOL einbeziehen soll. Für diesen (auch als "formellose IOL-Berechnung" bezeichneten) Ansatz sind nicht nur die genauen Baudaten der verwendeten Intraokularlinsen, sondern auch die vollständige Beschreibung der optischen Flächen im Auge notwendig. Insbesondere muss die Form der Hornhautvorder- und Rückfläche genau bekannt sein.

Schließlich wird das Problem der IOL-Berechnung nach refraktiver Hornhautchirurgie (z. B. RK, CK, PRK, und LASIK) immer weiter verbreitet. Das Grundproblem besteht bei diesen Fällen darin, dass die natürliche Beziehung zwischen den Radien der Hornhautvorder- und Hornhautrückfläche (das so genannte Gullstrand-Verhältnis), welches als Annahme in alle gängigen IOL-Berechnungsformeln implizit eingeht, durch den hornhautchirugischen Eingriff nachhaltig verändert wird. Darüber hinaus erzeugen Verfahren wie RK und CK, aber auch PRK- und LASIK-Eingriffe mit kleinen oder dezentrierten Behandlungszonen stark multifokale Hornhäute, deren Brechkraft nur bedingt durch eine Messung an einer Reihe von diskreten Messpunkten bestimmt werden kann.

Eine Reihe von Berechnungsansätzen für post-refraktive Augen ist in der Literatur beschrieben. Die im IOLMaster implementierte Haigis-L Formel nutzt den Umstand, dass das Keratometer des IOLMaster relativ achsnah misst, und liefert für Augen nach myoper LASIK im Mittel ausgezeichnete Ergebnisse.

Die Oculus Pentacam ermöglicht auf der Basis einer rotierenden Scheimpflugabbildung eine vollständige Charakterisierung der Hornhautvorder- und Hornhautrückfläche, sowie die Messung von Vorderkammertiefe und Linsendicke. Mithilfe des so genannten Holladay-Report ermöglicht sie die Bestimmung der Hornhaut-Gesamtbrechkraft auch von post-refraktiven Augen.

Die DE 198 12 297 C2 beschreibt ein Kombinationsgerät, mit welchem die Augenlänge, zentrale Hornhautradien sowie die Vorderkammertiefe des menschlichen Auges gemessen werden können. Die vorliegende Erfindung zielt darüber hinaus darauf ab, weitere Parameter im vorderen Augenabschnitt zu erfassen.

DE 299 13 601 U1, DE 299 13 602 U1, DE 299 13 603 U1 beschreiben eine Scheimpflugkamera, welche zur Darstellung des vorderen Augenabschnittes verwendet wird. Während die Literatur den Einsatz eines solchen Geräts im Zusammenhang mit der Bestimmung von Intraokularlinsen beschreibt, ist eine Augenlängenmessung mit dem Gerät nicht möglich. Demgegenüber zielen die hier beschriebenen Vorrichtungen und Verfahren darauf ab, alle benötigten Parameter mit einem Gerät zu bestimmen.

Das in US 6 634 751 dargelegte System verwendet ausdrücklich zwei separate Geräte zur Vermessung der Parameter im vorderen Augenabschnitt und zur Augenlängenmessung, und berechnet ausgehend von diesen Messungen eine Intraokularlinse. Im Gegensatz dazu werden in der vorliegenden Erfindung beide Messaufgaben vom selben Gerät erfüllt.

Ähnlich wie in der vorliegenden Erfindung wird in DE 198 52 331 wird ein Gerät vorgestellt, welches eine interferometrische Achslängenmessung mit einer zusätzlichen Messvorrichtung zur Erfassung zusätzlicher Parameter verbindet. Die zusätzliche Messvorrichtung ist jedoch immer ausdrücklich ein Placido-Ringsystem. Dieses Ringsystem ermöglicht jedoch nur die Vermessung der Hornhaut-Vorderfläche und ist zur Erfassung weiterer Strukturen im Vorderabschnitt, wie z. B. der Hornhaut-Rückfläche oder der natürlichen Linse ungeeignet. Im Gegensatz dazu ist in der vorliegenden Erfindung die zusätzliche Messvorrichtung in der Lage, mehrere Strukturen im Vorderabschnitt gleichzeitig zu erfassen. Dies geschieht erfindungsgemäß beispielsweise mithilfe von Spaltbeleuchtung in Verbindung mit einer Scheimpflug-Anordnung.

Die in der US 6,779,891 B1 beschriebene Lösung betrifft ein Kombinationsgerät zur berührungslosen Bestimmung von Achslänge (AL), Vorderkammertiefe (VKT) sowie Hornhautkrümmung (HHK) des Auges, insbesondere für die Berechnung und Auswahl einer zu implantierenden Intraokularlinse (IOL). Zur Vermessung der Achslänge verfügt die Lösung über ein Michelson- Interferometer, wobei die Messung beispielsweise dem aus der US 5,673,096 A bekannten Verfahren erfolgt. Zur Beobachtung des Auges und der entstehenden Reflexe wird ein Teil des vom Auge reflektierten Lichts mittels eines Achromaten über einen Spiegel auf eine CCD Kamera abgebildet. Zur Vermessung der Hornhautkrümmung HHK verfügt die Lösung über eine Keratometeranordnung, wobei die Messung analog der in der DD 251497 beschriebenen lösung erfolgt. Für die Bestimmung der Vorderkammertiefe VKT wird jedes Auge unter einem Winkel von ca. 33° mittels einer Spaltblende beleuchtet.

Auch die in der DE 198 57 001 A beschriebene Lösung betrifft ein Gerät zur berührungslosen Bestimmung der für die Auswahl der zu implantierenden Intraokularlinse IOL wichtigsten Parameter des Auges, wie Achslänge (AL), Vorderkammertiefe (VKT) und Hornhautkrümmung (HHK). Wie die in der zuvor beschriebenen Lösung basiert das Gerät ebenfalls auf einem Michelson- Interferometer, einer Keratometeranordnung, einer Spaltbeleuchtung und einer entsprechenden CCD-Kamera.

In der WO 2005/077256 A wird ein System zur Augenuntersuchung, insbesondere zur Bestimmung mehrerer Parameter des Auges vorgeschlagen. Dazu verfügt die beschriebene Lösung über Systeme zur Keratometrie, zur Kohärenz-Reflektometrie und zur Kohärenz-Interferometrie. Die gewünschten Parameter können somit entweder direkt gemessen oder aus den ermittelten Messwerten berechnet werden.

In dem Artikel "Micrometer-Scale Resolution Imaging of the anterior Eye in vivo with optical coherence Tomography",(ARCHIVES OF OPHTHALMOLOGY, Bd. 112, 1. Dezember 1994 (1994-12-01), Seiten 1584-1589) wird von J. A. Izatt und anderen ebenfalls zum Ausdruck gebracht, dass die optische Kohärenztomographie Potenzial als diagnostisches Werkzeug für Anwendungen in der kontaktlosen Biometrie hat und insbesondere auch zur Beurteilung, Identifizierung und Überwachung von intraokularen Massen und Tumoren und Aufklärung von Anomalien der Hornhaut, Iris und Linse Verwendung finden kann.

US 2005/0203422 A1 beschreibt ebenfalls ein Kombinationsgerät, bei dem alle Parameter außer denen der Hornhaut-Vorderfläche mittels interferometrischer Verfahren erfasst werden. Gegenüber der vorliegenden Erfindung bedeutet das aufgrund der benötigten Scanvorrichtung einen sehr hohen apparativen Aufwand was erhebliche Kostennachteile bedingt. In der vorliegenden Erfindung sollen die zusätzlichen Messfunktionen möglichst kostengünstig realisiert werden, was erfindungsgemäß durch die gleichzeitige Erfassung mehrerer Strukturen im Vorderabschnitt geschieht.

Der vorliegenden Erfindung liegt die Aufgabe zugrunde zusätzlich zu den üblicherweise verwendeten Größen Achslänge, Hornhaut-Vorderradien, Vorderkammertiefe und Limbusdurchmesser weitere Parameter des vorderen Augenabschnitts zur Berechnung der sphärischen Brechkraft von Intraokularlinsen in einem einzigen kompakten Gerät zu gewinnen. Dabei liegt besonderes Augenmerk auf der Gewinnung von Messdaten zur Berechnung der sphärischen Brechkraft von Intraokularlinsen nach früherem hornhautchirurgischem Eingriff. Insbesondere sollen axiale bzw. achsparallele Teilstrecken (z. B. Hornhautdicke, Linsendicke) und Radien (z. B. Hornhautvorder- und Hornhautrückradien in verschiedenen Meridianschnitten und verschiedenen optischen Zonen und Linsenvorder- und Linsenrückradien) ermittelt werden.

Mit dem erfindungsgemäßen Verfahren zur Augenvermessung werden die Achslänge, Hornhaut-Vorderradien und Vorderkammertiefe sowie weitere Parameter im vorderen Augenabschnitt erfasst, in dem mit Hilfe einer Steuereinheit und einer ersten Messvorrichtung die Achslänge mittels Kurzkohärenzinterferometrie und mit Hilfe der Steuereinheit und einer zusätzlichen Messvorrichtung mehrere Strukturen im Vorderabschnitt, wie Hornhaut, Vorderkammer und Linse mittels Spaltprojektion erfasst und die sphärische Brechkraft der IOL unter Berücksichtigung der gemessenen Teilstrecken des Auges und ihrer unterschiedlichen Brechzahlen unter Verwendung einer IOL-Berechnungsformel und empirisch gewonnenen Konstanten oder mit Hilfe eines Raytracing-Verfahrens berechnet werden, wobei die zusätzliche Messvorrichtung über mindestens eine, axial zur Achse des Auges angeordnete Beleuchtungseinheit und mindestens eine, geneigt zur Achse des Auges angeordnete Bildaufnahmeeinheit verfügt, deren Sensorchip zum Zeitpunkt der Bildaufnahme mit der beleuchteten Ebene im Auge die Scheimpflugbedingung erfüllt.

Dabei ist das Achslängenmessverfahren vorzugsweise ein (im Zeit- oder Frequenzraum arbeitendes) optisches Kohärenzinterferometer (PCI, OCT).

Das Schnittbildverfahren ist vorzugsweise ein optisches Verfahren, das eine strukturierte Beleuchtung z. B eine Spaltbeleuchtung und mindestens eine Kamera nutzt. Dabei erfüllen die beleuchtete Ebene im Auge, die Hauptebene der Geräteoptik und die Kameraebene vorzugsweise die Scheimpflug-Bedingung. Die Spaltbeleuchtung wird vorzugsweise von LEDs erzeugt, und kann in einer Ausführungsform aus einer Anordnung diskreter (kollimierter) Strahlen bestehen.

Das erfindungsgemäße Verfahren zur Achslängenmessung betrifft eine Lösung zur Messung geometrischer, zur Berechnung von Intraokularlinsen erforderlichen Parameter im Auge. Die Ermittlung von Teilstrecken, sowie der Parameter der vorderen Augenabschnitte ermöglicht eine genauere Berechnung der mittels OCT gewonnenen Augenlänge, da bei der Umrechnung von optischer in geometrische Weglänge die Brechungsindizes der einzelnen Augenabschnitte verwendet werden können. Durch die Bestimmung zusätzlicher Teilstrecken Parameter der vorderen Augenabschnitte können selbst nach einer refraktiven, chirurgischen Behandlung, bei der die natürliche Beziehung zwischen den Radien der Hornhautvorder- und Hornhautrückfläche (das so genannte Gullstrand-Verhältnis), welches als Annahme in alle gängigen IOL-Berechnungsformeln implizit eingeht, durch den hornhautchirurgischen Eingriff nachhaltig verändert wird, die Berechnung der IOL mit hohe Genauigkeit erreicht werden.

Die Erfindung wird nachfolgend anhand von Ausführungsbeispielen näher beschrieben. Dazu zeigen:
- Figur 1:: den Prinzipaufbau eines IOLMaster (Stand der Technik),
- Figur 2:: die Kombination einer PCI-Achslängenmessung mit einem axialen Spaltprojektor und einer geneigt angeordneten Bildaufnahmeeinheit,
- Figur 3:: eine azimuthale Anordnung der Bildaufnahmeoptik mit entsprechender Spaltorientierung,
- Figur 4:: eine azimuthale Anordnung mehrerer Bildaufnahmeoptiken mit entsprechenden Spaltorientierungen,
- Figur 5:: die Kombination PCI-Achslängenmessung mit einer ortsfesten, axialen Bildaufnahmeeinheit und drei ortsfesten, geneigten Spaltprojektoren,
- Figur 6:: eine azimuthale Anordnung eines Spaltprojektors bei axial angeordneter Bildaufnahmeoptik und
- Figur 7:: eine azimuthale Anordnung mehrerer Spaltprojektoren bei axial angeordneter Bildaufnahmeoptik.

Bei dem erfindungsgemäßen Verfahren zur Augenvermessung wird die Achslänge, Hornhaut-Vorderradien und Vorderkammertiefe sowie weitere Parameter im vorderen Augenabschnitt erfasst, in dem mit Hilfe einer Steuereinheit und einer ersten Messvorrichtung die Achslänge mittels Kurzkohärenzinterferometrie und mit Hilfe der Steuereinheit und einer zusätzlichen Messvorrichtung mehrere Strukturen im Vorderabschnitt, wie Hornhaut, Vorderkammer und Linse mittels Spaltprojektion erfasst und die sphärische Brechkraft der IOL unter Berücksichtigung der gemessenen Teilstrecken des Auges und ihrer unterschiedlichen Brechzahlen unter Verwendung einer IOL-Berechnungsformel und empirisch gewonnenen Konstanten oder mit Hilfe eines Raytracing-Verfahrens berechnet werden, wobei die zusätzliche Messvorrichtung über mindestens eine, axial zur Achse des Auges angeordnete Beleuchtungseinheit und mindestens eine, geneigt zur Achse des Auges angeordnete Bildaufnahmeeinheit verfügt, deren Sensorchip zum Zeitpunkt der Bildaufnahme mit der beleuchteten Ebene im Auge die Scheimpflugbedingung erfüllt.

Dabei basiert die Achslängenmessung auf der Kurzkohärenzinterferometrie und ist auch geeignet axiale und/oder achsparallele Teilstrecken im Vorderabschnitt, Hornhautdicke, Vorderkammertiefe und Linsendicke, wie zentral, para-zentral, mittelperipher und/oder peripher zu bestimmen, wobei achsparallele Teilstrecken im Vorderabschnitt durch laterale Ablenkung des Messstrahls oder laterale Verschiebung der Messvorrichtung erfasst werden. Axiale Teilstrecken, achsparallele Teilstrecken, laterale Abstände, Krümmungsradien und/oder Brechungswinkel werden hierbei mit Hilfe der zusätzlichen Messvorrichtung bestimmt, wobei diese und über mindestens eine Beleuchtungseinheit zur Erzeugung von strukturierter Beleuchtung verfügt. Von der Beleuchtungseinheit wird eine strukturierte Beleuchtung in Form einer Spalt- oder Streifenprojektion mittels, auf Halbleiterverbindungen basierender, Licht emittierender Dioden oder auch eines oder mehrerer opto-elektronische Lichtmodulatoren beispielsweise Mikrodisplays erzeugt. Während als auf Halbleiterverbindungen basierende, Licht emittierende Dioden beispielsweise LED's (light emitting diode) oder OLED's (organic light emitting diode) verwendet werden, kommen als opto-elektronische Lichtmodulatoren beispielsweise Mikrodisplays vom DMD-Typ (digital micromirror device) bzw. reflektierende Mikrodisplays vom LCOS-Typ (liquid crystal on silicon) zum Einsatz.

Bei dem erfindungsgemäßen Verfahren zur Augenvermessung kann die strukturierte Beleuchtung auch in Form einer Reihe fokaler Bündel erzeugt werden, wobei die Steuereinheit aus dem von der Bildaufzeichnungseinheit erfassten Bild die Brechungswinkel der fokalen Bündel ermittelt, um daraus die Brechkräfte der brechenden Flächen zu berechnen.

Mit Hilfe der von einer einzelnen Beleuchtungseinheit erzeugten, zeitlich veränderlichen Spaltbeleuchtung können unterschiedliche meridionale Schnitten durch den Vorderabschnitt des Auges erzeugt und von einer Bildaufzeichnungseinheit aufgezeichnet werden. Dabei sind die Bildaufzeichnungseinheit und/oder die Beleuchtungseinheit geneigt zur Achse des Auges angeordnet und können vorzugsweise um diese rotieren. Es ist aber auch möglich, mehrere ortsfeste Beleuchtungseinheiten geneigt zur Achse des Auges anzuordnen und die Spaltbeleuchtung in unterschiedlichen meridionalen Schnitten unter Veränderung der Fixationsrichtung des Patientenauges zu erzeugen.

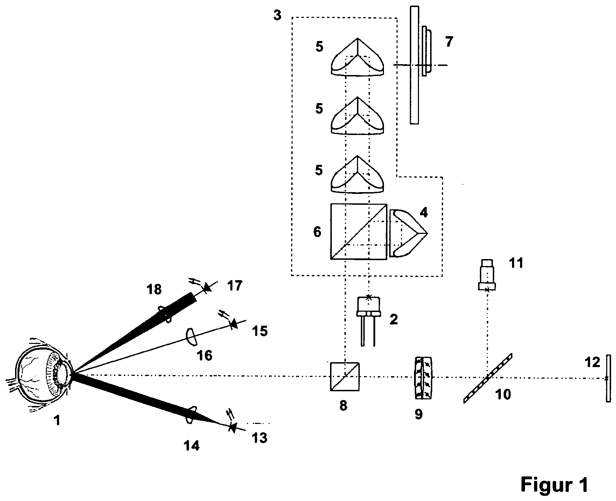

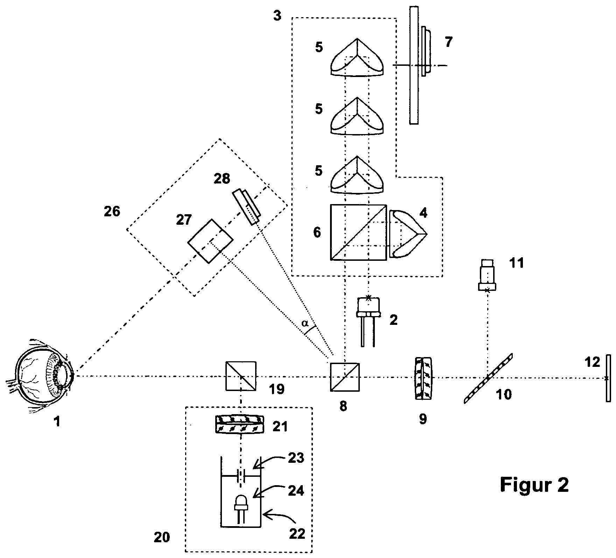

Hierzu zeigt **Figur 2** eine mögliche Vorrichtung zur Durchführung des erfindungsgemäßen Verfahrens, wobei die PCI-Achslängenmessung (IOLMaster nach **Figur 1**) mit einer axialen Spaltprojektion und einer geneigt angeordneten Bildaufnahme kombiniert wurde. Dabei erfolgt die Achslängenmessung wie üblich zentral entlang der Fixierrichtung des Patienten. Zusätzlich erfolgt eine Spaltbeleuchtung mittels Spaltprojektor **20** zentral zur Augenachse. Das von der LED-Beleuchtung **24** und dem Spalt **23** erzeugte Spaltbild wird über einen Strahlteilerwürfel **19** in das Auge **1** projiziert. Die Bildaufzeichnung erfolgt über eine, aus Abbildungsoptik **27** und Kamera **28** bestehende, zur Augenachse geneigt angeordnete Bildaufnahmeeinheit **26.**

In einer ersten Ausführungsform ist die, aus LED-Beleuchtung **24** und Spalt **23** bestehende Spaltleuchte **22** drehbar ausgeführt, so dass das erzeugte Spaltbild um die Augenachse rotieren kann. Zur Aufnahme der im Auge **1** erzeugten Spaltbilder ist die geneigt zur Augenachse positionierte Bildaufnahmeeinheit **26** so ausgebildet, dass diese um die Augenachse rotiert. Vorzugsweise verfügt die Kamera **28** der Bildaufzeichnungseinheit **26** über einen Sensorchip, wie einen CCD- oder CMOS-Sensor, welcher zum Zeitpunkt der Bildaufnahme mit der beleuchteten Ebene im Auge **1** die Scheimpflugbedingung annähernd oder genau erfüllt. Dabei wird der Vorderabschnitt des Auges **1** in verschiedenen Ebenen mit dem vom Spaltprojektor **20** erzeugten Spaltbild abgetastet und zu unterschiedlichen Zeitpunkten mit der Kamera **28** zur späteren Auswertung aufgenommen und/oder gespeichert.

In einer zweiten Ausführungsform sind zur Aufnahme der im Auge **1** erzeugten Spaltbilder mehrere, geneigt zur Augenachse positionierte Bildaufnahmeeinheit **26** ortsfest positioniert. Auch hier ist die aus LED-Beleuchtung **24** und Spalt **23** bestehende Spaltleuchte **22** drehbar ausgeführt, so dass das erzeugte Spaltbild um die Augenachse rotieren kann. Vorzugsweise verfügen auch hier die Kameras **28** der Bildaufzeichnungseinheiten **26** über Sensorchips, welche zum Zeitpunkt der Bildaufnahme mit der beleuchteten Ebene im Auge **1** die Scheimpflugbedingung annähernd oder genau erfüllen. Der Vorderabschnitt des Auges **1** wird auch hier in verschiedenen Ebenen mit dem vom Spaltprojektor **20** erzeugten Spaltbild abgetastet und mit der Mehrzahl von Kameras **28** zur späteren Auswertung aufgenommen und/oder gespeichert.

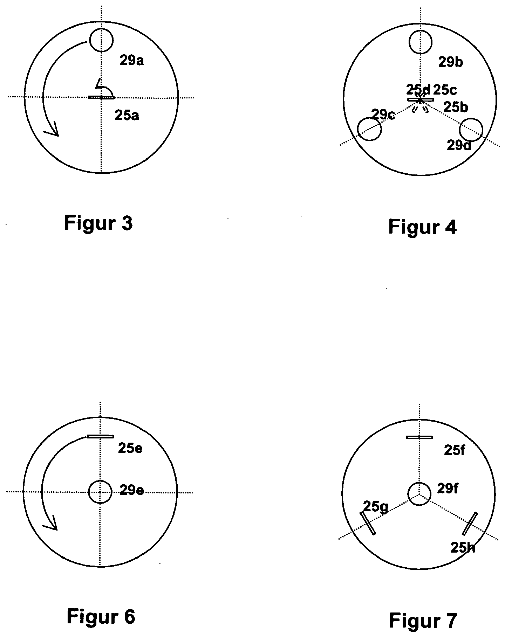

Zur besseren Veranschaulichung und zur Gegenüberstellung dieser beiden Ausführungsformen zeigen **Figur 3** eine azimuthale Anordnung der Bildaufnahmeoptik und entsprechende Spaltorientierung für die erste und **Figur 4** für die zweite Ausführungsform. Während in **Figur 3** sowohl das Abbild **25a** des Spaltes **23** als auch der als Kreis dargestellte Abbildungsstrahlengang **29a** der Bildaufzeichnungseinheit **26** um die Augenachse rotieren und eine beliebige Anzahl von Spaltbildern unter verschiedenen Winkeln aufgenommen werden können, rotiert in **Figur 4** zwar das Abbild des Spaltes **23,** kann aber durch ortsfest positionieren Bildaufnahmeeinheiten **26** nur als Abbilder **25b, 25c** und **25d** aufgenommen werden. Mit den Bezugszeichen **29b, 29c** und **29d** sind hier die Abbildungsstrahlengänge der ortsfest positionierten Bildaufnahmeeinheiten **26** bezeichnet.

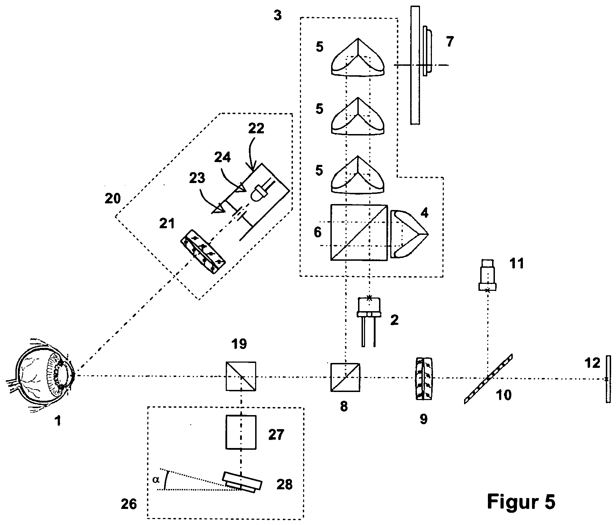

**Figur 5** zeigt hierzu eine weitere Vorrichtung zur Durchführung des erfindungsgemäßen Verfahrens als Kombination von PCI-Achslängenmessung mit axialer Bildaufzeichnung und mindestens einer geneigt angeordneten Spaltprojektion. Hierzu wird entweder ein um die Augenachse rotierender oder mehrere, ortfest angeordnete Spaltprojektoren **20** verwendet.

In einer dritten Ausführungsform des erfindungsgemäßen Verfahrens wird die zur Bildaufzeichnung vorhandene Bildaufnahmeeinheit **26** entlang dieser Achse angeordnet und beispielsweise über den Strahlteilerwürfel **19** eingekoppelt. Zur Erzeugung von Spaltbildern im Auge **1** ist mindestens ein, geneigt zur Augenachse positionierter Spaltprojektor **20** angeordnet. Somit erfolgt die Spaltabbildung auf das Auge **1** unter mindestens einem definierten Winkel zur Augenachse. Zur Abbildung meridionaler Schnittbilder durch die Vorderkammer muss der Patient auf einen einstellbaren Fixationspunkt blicken. Soll eine Reihe von Spaltbildern unter verschiedenen Azimutwinkeln aufgenommen werden, ohne dafür den Spaltprojektor **20** bzw. die Spaltleuchte **22** zu bewegen, ist das sequentielle Fixieren des Patienten auf eine Anzahl von verschiedenen Fixationspunkten erforderlich. Vorzugsweise erfüllen die vom Spaltprojektor **20** beleuchtete Ebenen im Auge **1** zu unterschiedlichen Zeitpunkten mit dem Sensorchip der Bildaufnahmeeinheit **26** die Scheimpflugbedingung annähernd oder genau.

In einer vierten Ausführungsform des erfindungsgemäßen Verfahrens wird die zur Bildaufzeichnung vorhandene Bildaufnahmeeinheit **26** entlang dieser Achse ortsfest angeordnet und beispielsweise über den Strahlteilerwürfel **19** eingekoppelt. Zur Erzeugung von Spaltbildern im Auge **1** sind eine Mehrzahl, geneigt zur Augenachse positionierter Spaltprojektoren **20** angeordnet. Die Spaltabbildung auf das Auge **1** erfolgt somit unter mehreren definierten Winkeln zur Augenachse. Vorzugsweise erfüllen die von den Spaltprojektoren **20** beleuchteten Ebenen im Auge **1** zu unterschiedlichen Zeitpunkten mit dem Sensorchip der Bildaufnahmeeinheit **26** die Scheimpflugbedingung annähernd oder genau.

Zur besseren Veranschaulichung und zur Gegenüberstellung dieser beiden Ausführungsformen zeigen **Figur 6** eine azimuthale Anordnung der Bildaufnahmeoptik und entsprechende Spaltorientierung für die dritte und **Figur 7** für die vierte Ausführungsform. Während bei feststehendem Abbildungsstrahlengang **29e** der Bildaufzeichnungseinheit **26** in **Figur 6** das Abbild **25e** des Spaltes **23** rotiert und so eine beliebige Anzahl von Spaltbildern unter verschiedenen Winkeln aufgenommen werden kann, sind in **Figur 4** sowohl die Abbilder **25f, 25g** und **25h** des Spaltes **23** als auch der als Kreis dargestellte Abbildungsstrahlengang **29f** der Bildaufzeichnungseinheit **26** ortsfest angeordnet, so dass nur drei Spaltbildern aufgenommen werden können.

Die nicht dargestellte Steuereinheit verfügt neben Mitteln zur Erfassung und Auswertung der für die Achslängenmessung erforderlichen Messwerte auch über Mittel zur Erfassung und Auswertung der für eine Triangulation am Auge erforderlichen Winkel- und Abstandsverhältnisse, wobei aus den von der Bildaufzeichnungseinheit gelieferten Bildern der vorderen Augenabschnitte bei strukturierter Beleuchtung Teilstrecken, Radien und/oder Brechungswinkel bestimmt werden.

Zusätzlich ist es vorteilhaft, wenn die Steuereinheit über Mittel zur Durchführung dynamischer Messmodi, wie sequentielle Bildaufnahme, Differenzbildaufnahme o. ä. verfügt und sie in der Lage ist, die entsprechende Messungen simultan durchzuführen und auszuwerten, wobei sich die Ergebnisse der einen Messung die Durchführung der jeweils anderen Messung beeinflussen können.

In Auswertung aller gewonnenen Parameter wird von der Steuereinheit mit Hilfe von Linsenberechnungs-Formeln und empirisch gewonnenen Konstanten oder mit Hilfe eines Raytracing-Verfahrens die benötigte sphärische Brechkraft einer Intraokularlinse berechnet. Zusätzlich zur sphärischen Brechkraft wird neben der benötigten Zylinder-Brechkraft auch ein geeigneter Asphärizitätswert für eine Intraokularlinse berechnet.

Mit der erfindungsgemäßen Vorrichtung und dem Verfahren zur Augenvermessung wird eine Lösung zur Verfügung gestellt, mit der zusätzlich zu den üblicherweise verwendeten Größen Achslänge, Hornhaut-Vorderradien, Vorderkammertiefe und Limbusdurchmesser weitere Parameter des vorderen Augenabschnitts zur Berechnung von Intraokularlinsen insbesondere nach hornhautchirurgischen Eingriffen, in einem einzigen kompakten Gerät gewonnen werden können. Besonders vorteilhaft sind die Bestimmung axialer bzw. achsparalleler Teilstrecken (z. B. Hornhautdicke, Linsendicke) und Radien (z. B. Hornhautvorder- und Hornhautrückradien in verschiedenen Meridianschnitten und verschiedenen optischen Zonen und Linsenvorder- und Linsenrückradien). Dadurch ist die Bestimmung aller für die Berechnung und Auswahl einer IOL erforderlichen Parameter mit der erforderlichen Genauigkeit und möglichst geringem apparativem Aufwand möglich.

Die Kenntnis der Teilstrecken ermöglicht eine genauere Berechnung der mittels PCl gewonnenen Augenlänge, da bei der Umrechnung von optischer Weglange in geometrische Weglange die Brechungsindizes der einzelnen Augenabschnitte verwendet werden können.

### Bezugszeichenliste

- 1: Auge
- 2: Laserdiode
- 3: Michelson-Interferometer, *bestehend aus:*
- 4: Tripelprisma
- 5: Tripelprisma (verschiedene Positionen)
- 6: Strahlteilerwürfel
- 7: Wegmesssystem
- 8: Strahlteilerwürfel
- 9: Achromat
- 10: Strahlteiler
- 11: Empfänger
- 12: Kamera
- 13, 15: Lichtquelle für Keratometer
- 14, 16: Beleuchtungsoptik für Keratometer
- 17: Lichtquelle für VKT-Messung
- 18: Beleuchtungsoptik für VKT-Messung
- 19: Strahlteilerwürfel
- 20: Spaltprojektor, *bestehend aus:*
- 21: Projektionsoptik
- 22: drehbare Spaltleuchte, *bestehend aus:*
- 23: Spalt
- 24: LED-Beleuchtung
- 25: Abbilder des Spaltes 23
- 26: Bildaufnahmeeinheit, *bestehend aus:*
- 27: Abbildungsoptik
- 28: Kamera
- 29: Abbildungsstrahlengänge der Bildaufnahmeeinheit 26

## Patentansprüche

1. Verfahren zur Augenvermessung, mit dem die Achslänge, Hornhaut-Vorderradien und Vorderkammertiefe sowie weitere Parameter im vorderen Augenabschnitt erfasst werden, in dem mit Hilfe einer Steuereinheit und einer ersten Messvorrichtung die Achslänge mittels Kurzkohärenzinterferometrie und mit Hilfe der Steuereinheit und einer zusätzlichen Messvorrichtung mehrere Strukturen im Vorderabschnitt, wie Hornhaut, Vorderkammer und Linse mittels Spaltprojektion erfasst und die sphärische Brechkraft der IOL unter Berücksichtigung der gemessenen Teilstrecken des Auges und ihrer unterschiedlichen Brechzahlen unter Verwendung einer IOL-Berechnungsformel und empirisch gewonnenen Konstanten oder mit Hilfe eines Raytracing-Verfahrens berechnet werden, wobei die zusätzliche Messvorrichtung über mindestens eine, axial zur Achse des Auges angeordnete Beleuchtungseinheit und mindestens eine, geneigt zur Achse des Auges angeordnete Bildaufnahmeeinheit verfügt, deren Sensorchip zum Zeitpunkt der Bildaufnahme mit der beleuchteten Ebene im Auge die Scheimpflugbedingung erfüllt.

2. Verfahren nach Anspruch 1, wobei auch axiale und/oder achsparallele Teilstrecken im Vorderabschnitt, wie zentrale, zonale und/oder periphere Hornhautdicke, Vorderkammertiefe und Linsendicke, mittels Kurzkohärenzinterferometrie bestimmt werden.

3. Verfahren nach Anspruch 2, wobei achsparallele Teilstrecken im Vorderabschnitt durch laterale Ablenkung des Messstrahls oder laterale Verschiebung der Messvorrichtung erfasst werden.

4. Verfahren nach Anspruch 2, bei welchem axiale Teilstrecken, achsparallele Teilstrecken, laterale Abstände, Krümmungsradien und/oder Brechungswinkel zu mithilfe der zusätzlichen Messvorrichtung bestimmt werden, wobei diese und über mindestens eine Beleuchtungseinheit zur Erzeugung von strukturierter Beleuchtung verfügt.

5. Verfahren nach Anspruch 4, wobei die strukturierte Beleuchtung als Spaltbeleuchtung oder Streifenprojektion ausgebildet ist, und mittels herkömmlicher Leuchtdioden (LED) oder Leuchtdioden auf der Basis organischer Materialien (OLED) erzeugt wird.

6. Verfahren nach Anspruch 5, wobei die strukturierte Beleuchtung als Spaltbeleuchtung oder Streifenprojektion ausgebildet ist, und mittels eines oder mehrerer Flächenlichtmodulatoren, wie digitaler Mikrospiegel-Arrays (DMD) oder Flüssigkristall-Lichtmodulatoren (LCOS) erzeugt wird.

7. Verfahren nach Anspruch 5, wobei die strukturierte Beleuchtung in Form einer Reihe fokaler Bündel erzeugt wird und aus dem von der Bildaufzeichnungseinheit erfassten Bild von der Steuereinheit die Brechungswinkel der fokalen Bündel ermittelt werden, um daraus die Brechkräfte der brechenden Flächen zu berechnen.

8. Verfahren nach Anspruch 5, wobei eine einzelne Beleuchtungseinheit zeitlich veränderlich Spaltbeleuchtung in unterschiedlichen meridionalen Schnitten durch den Vorderabschnitt erzeugt.

9. Verfahren nach Anspruch 5, wobei mehrere Beleuchtungseinheiten geneigt zur Achse des Auges angeordnet sind und Spaltbeleuchtung in unterschiedlichen meridionalen Schnitten unter Veränderung der Fixationsrichtung des Patientenauges erzeugen.

10. Verfahren nach Anspruch 9, wobei die Bildaufnahmeeinheit und/oder die Beleuchtungseinheit geneigt zur Achse des Auges angeordnet sind und vorzugsweise um diese rotieren können.

11. Verfahren nach Anspruch 10, wobei eine Mehrzahl von ortsfesten Bildaufnahmeeinheiten vorhanden ist, die zu unterschiedlichen Zeitpunkten mit der jeweils beleuchteten Ebene im Auge die Scheimpflugbedingung erfüllen.

12. Verfahren nach Anspruch 10, wobei eine Mehrzahl von ortsfesten Beleuchtungseinheiten vorhanden ist, deren beleuchtete Ebenen im Auge zu unterschiedlichen Zeitpunkten mit dem Sensorchip die Scheimpflugbedingung erfüllen.

13. Verfahren nach Anspruch 10, wobei genau eine Beleuchtungseinheit vorhanden ist, deren strukturierte Beleuchtung den Vorderabschnitt in verschiedenen Ebenen abtastet und dabei zu unterschiedlichen Zeitpunkten mit einem Sensorchip die Scheimpflugbedingung erfüllt.

14. Verfahren nach Anspruch 1, wobei die Blickrichtung des Patienten über eine einstellbare Fixiereinheit gelenkt wird, die vorzugsweise in Form, Farbe, Intensität und Position variiert werden kann.

15. Verfahren nach Anspruch 14, wobei der Akkomodationszustand des Patientenauges mithilfe der Fixiereinheit zusätzlich beeinflusst werden kann.

16. Verfahren nach Anspruch 1, wobei die für eine Triangulation am Auge erforderlichen Winkel- und Abstandsverhältnisse erfasst und ausgewertet werden.

17. Verfahren nach Anspruch 1, wobei aus den gewonnenen Daten zusätzlich zur sphärischen Brechkraft die benötigte Zylinder-Brechkraft einer Intraokularlinse berechnet wird.

18. Verfahren nach Anspruch 1, wobei aus den gewonnenen Daten zusätzlich zur Bestimmung der benötigten sphärischen Brechkraft ein geeigneter Asphärizitätswert für eine Intraokularlinse berechnet wird.

## Claims

1. Method for measuring the eye, by means of which the axial length, anterior corneal radii and anterior chamber depth and further parameters in the front eye segment are captured, in which the axial length is captured by means of low coherence interferometry with the aid of a control unit and a first measuring appliance and a plurality of structures in the anterior segment, such as cornea, anterior chamber and lens, are captured by means of a slit projection with the aid of the control unit and an additional measuring appliance and the spherical refractive power of the IOL is calculated taking into account the measured portions of the eye and their different refractive indices using an IOL calculation formula and empirically obtained constants or with the aid of a ray tracing method, wherein the additional measuring appliance comprises at least one illumination unit that is arranged axially in relation to the axis of the eye and at least one image recording unit that is arranged inclined in relation to the axis of the eye, the sensor chip of said at least one image recording unit satisfying the Scheimpflug condition with the illuminated plane in the eye at the instant of the image recording.

2. Method according to Claim 1, wherein axial and/or axially parallel portions in the anterior segment, such as central, zonal and/or peripheral corneal thickness, anterior depth and lense thickness are also determined by means of short coherence interferometry.

3. Method according to Claim 2, wherein axially parallel portions in the anterior segment are captured by a lateral deviation of the measurement beam or a lateral displacement of the measuring appliance.

4. Method according to Claim 2, in which axial portions, axially parallel portions, lateral distances, radii of curvature and/or refractive angles in relation to are determined with the aid of the additional measuring appliance, wherein the latter and has at least one lighting unit for producing structured illumination.

5. Method according to Claim 4, wherein the structured illumination is embodied as slit illumination or structured light projection and produced by means of conventional light-emitting diodes (LEDs) or light emitting diodes on the basis of organic materials (OLEDs).

6. Method according to Claim 5, wherein the structured illumination is embodied as slit illumination or structured light projection and produced by means of one or more spatial light modulators, such as digital micromirror devices (DMDs) or liquid-crystal light modulators (LCOSs).

7. Method according to Claim 5, wherein the structured illumination is produced in the form of a sequence of focal beams and the refractive angles of the focal beams are ascertained by the control unit from the image captured by the image-recording unit in order to calculate the refractive powers of the refractive surfaces.

8. Method according to Claim 5, wherein a single lighting unit produces temporally changeable slit illumination in different meridional sections through the anterior segment.

9. Method according to Claim 5, wherein a plurality of lighting units are arranged in a manner inclined in relation to the axis of the eye and produce a slit illumination in different meridional sections by changing the fixation direction of the patient's eye.

10. Method according to Claim 9, wherein the image-recording unit and/or the lighting unit are arranged in a manner inclined in relation to the axis of the eye and preferably able to rotate about the latter.

11. Method according to Claim 10, wherein a plurality of stationary image recording units that satisfy the Scheimpflug condition with the respectively illuminated plane in the eye at different instants are present.

12. Method according to Claim 10, wherein a plurality of stationary lighting units whose illuminated planes in the eye satisfy the Scheimpflug condition with the sensor chip at different instants are present.

13. Method according to Claim 10, wherein there is exactly one lighting unit, the structured illumination of which scans the anterior segment in various planes and, in the process, satisfies the Scheimpflug condition with a sensor chip at different instants.

14. Method according to Claim 1, wherein the direction of view of the patient is guided via an adjustable fixing unit, the form, colour, intensity and position of which can preferably be varied.

15. Method according to Claim 14, wherein the accommodation state of the patient's eye additionally can be influenced with the aid of the fixing unit.

16. Method according to Claim 1, wherein the angle and distance relationships that are required for triangulation on the eye are captured and evaluated.

17. Method according to Claim 1, wherein the required cylindrical refractive power of an intraocular lens, in addition to the spherical refractive power, is calculated from the obtained data.

18. Method according to Claim 1, wherein, in addition to determining the required spherical refractive power, of a suitable asphericity value for an intraocular lens is calculated from the obtained data.

## Revendications

1. Procédé de mesure de l'oeil, au moyen duquel on détecte la longueur axiale, les rayons cornéens antérieurs et la profondeur de la chambre antérieure ainsi que d'autres paramètres de la section antérieure de l'oeil, dans lequel, à l'aide d'une unité de commande et d'un premier dispositif de mesure, on détecte la longueur axiale par interférométrie à faible longueur de cohérence et on détecte plusieurs structures, telles que la cornée, la chambre antérieure et le cristallin, dans la section antérieure à l'aide de l'unité de commande et d'un dispositif de mesure supplémentaire par projection à travers une fente et on calcule le pouvoir de réfraction sphérique de l'IOL en tenant compte des segments mesurés de l'oeil et de leurs différents indices de réfraction par utilisation d'une formule de calcul d'IOL et de constantes obtenues empiriquement ou à l'aide d'un procédé de tracé de rayons, dans lequel le dispositif de mesure supplémentaire comporte au moins une unité d'éclairage disposée axialement par rapport à l'axe de l'oeil et au moins une unité d'acquisition d'image disposée de manière inclinée par rapport à l'axe de l'oeil, dont la puce de capteur, par rapport à l'instant d'acquisition d'image, satisfait à la condition de Scheimpflug avec le plan éclairé dans le oeil.

2. Procédé selon la revendication 1, dans lequel des segments axiaux et/ou parallèles à l'axe dans la section antérieure, tels que l'épaisseur cornéenne centrale, zonale et/ou périphérique, la profondeur de la chambre antérieure et l'épaisseur du cristallin, sont également déterminés par interférométrie à faible longueur de cohérence.

3. Procédé selon la revendication 2, dans lequel des segments parallèles à l'axe sont détectés dans la section antérieure par déviation latérale du faisceau de mesure ou par décalage latéral du dispositif de mesure.

4. Procédé selon la revendication 2, dans lequel des segments axiaux, des segments parallèles à l'axe, des espacements latéraux, des rayons de courbure et/ou des angles de réfraction relatifs à sont déterminés à l'aide du dispositif de mesure supplémentaire, dans lequel celui-ci et comporte au moins une unité d'éclairage destinée à générer un éclairage structuré.

5. Procédé selon la revendication 4, dans lequel l'éclairage structuré est réalisé sous la forme d'un éclairage par fente ou par projection de franges, et est généré par des diodes électroluminescentes classiques (LED) ou des diodes électroluminescentes à base de matériaux organiques (OLED).

6. Procédé selon la revendication 5, dans lequel l'éclairage structuré sous la forme est réalisé sous la forme d'un éclairage par fente ou par projection de franges, et est généré au moyen d'un ou plusieurs modulateurs spatiaux de lumière, tels que des réseaux de micromiroirs numériques (DMD) ou des modulateurs spatiaux de lumière à cristaux liquides (LCOS).

7. Procédé selon la revendication 5, dans lequel l'éclairage structuré est généré sous la forme d'une rangée de faisceaux focaux et les angles de réfraction des faisceaux focaux sont déterminés par l'unité de commande à partir de l'image détectée par l'unité d'acquisition d'image afin de calculer à partir de ceux-ci les pouvoirs de réfraction des surfaces réfringentes.

8. Procédé selon la revendication 5, dans lequel une unité d'éclairage individuelle génère à travers la section antérieure un éclairage par fente variant dans le temps dans des sections méridionales différentes.

9. Procédé selon la revendication 5, dans lequel plusieurs unités d'éclairage sont disposées de manière inclinée par rapport à l'axe de l'oeil et génèrent un éclairage par fente dans des sections méridionales différentes en modifiant la direction de fixation de l'oeil du patient.

10. Procédé selon la revendication 9, dans lequel l'unité d'acquisition d'image et/ou l'unité d'éclairage est/sont disposée(s) de manière inclinée par rapport à l'axe de l'oeil et peut/peuvent de préférence tourner autour de celui-ci.

11. Procédé selon la revendication 10, dans lequel il est prévu une pluralité d'unités d'acquisition d'image fixes qui satisfont à différents instants à la condition de Scheimpflug avec le plan respectivement éclairé dans l'oeil.

12. Procédé selon la revendication 10, dans lequel il est prévu une pluralité d'unités d'éclairage fixes, dont les plans éclairés dans l'oeil satisfont à différents instants à la condition de Scheimpflug avec la puce de capteur.

13. Procédé selon la revendication 10, dans lequel il est prévu exactement une unité d'éclairage, dont l'éclairage structuré balaye la section antérieure dans des plans différents et satisfait ainsi à différents instants à la condition de Scheimpflug avec une puce de capteur.

14. Procédé selon la revendication 1, dans lequel la direction du regard du patient est orientée par l'intermédiaire d'une unité de fixation réglable, dont la forme, la couleur, l'intensité et la position peuvent de préférence être amenées à varier.

15. Procédé selon la revendication 14, dans lequel l'état d'accommodation de l'oeil du patient peut être influencé de façon supplémentaire à l'aide de l'unité de fixation.

16. Procédé selon la revendication 1, dans lequel les rapports d'angles et de distances nécessaires pour une triangulation sur l'oeil sont détectés et évalués.

17. Procédé selon la revendication 1, dans lequel le pouvoir de réfraction cylindrique nécessaire d'une lentille intraoculaire est calculé à partir des données obtenues en plus du pouvoir de réfraction sphérique.

18. Procédé selon la revendication 1, dans lequel une valeur d'asphéricité appropriée est calculée pour une lentille intraoculaire à partir des données obtenues en plus de la détermination du pouvoir de réfraction sphérique nécessaire.
